# EUROPEAN PATENT APPLICATION

(11) **EP 4 250 719 A1**
(43) Date of publication of application: **27.09.2023**
(21) Application number: 23163576.4
(22) Date of filing: 22.03.2023
(51) Int. Cl.: H04N 7/15, H04N 7/14, H04L 65/1094, H04L 65/403, G16H 80/00

(54) **USE OF AUDIO AND/OR VIDEO IN PATIENT CARE**

(30) Priority: 23.03.2022 US 202263269802 P
(71) Applicant: Hill-Rom Services, Inc., Batesville, IN 47006 (US)
(72) Inventor: MEYERSON, Craig M., Batesville, IN 47006 (US); RIBBLE, David Lance, Batesville, IN 47006 (US); SHIRLEY, Daniel, Batesville, IN 47006 (US); SUAREZ, Carlos Andres, Batesville, IN 47006 (US); WOLFE, Gene J., Batesville, IN 47006 (US)
(74) Representative: Bauer, Dustin

(57) **Abstract**

An example method for delivery of patient information through audio and/or video can include: capturing audio and/or video from a caregiver; receiving identification of a patient from the caregiver; receiving authorization to deliver the audio and/or video in association with providing care for the patient; and delivering the audio and/or the video.

## Description

As the need for healthcare rises, the time spent by caregivers in patient care becomes even more valuable. Caregivers are continually asked to become more efficient in providing that care. This can include requirements to see additional patients in a given amount of time. The result is additional pressure on the caregivers, as the healthcare system already works at a perceived high level of efficiency.

In general terms, the present disclosure relates to the use of audio and/or video by caregivers to increase efficiencies in patient care. Various aspects are described in this disclosure, which include, but are not limited to, the following aspects.

In one aspect, an example method for delivery of patient information through audio and/or video can include: capturing audio and/or video from a caregiver; receiving identification of a patient from the caregiver; receiving authorization to deliver the audio and/or video in association with providing care for the patient; and delivering the audio and/or the video.

In another aspect, an example method for initiating a workflow for a patient can include: receiving a trigger event; upon receiving the trigger event, monitor for a command from a caregiver of the patient; and upon receiving the command, initiate the workflow associated with the command.

In yet another aspect, an example method for conducting a video conference associated with care of a patient can include: initiating the video conference on a first device; identifying at least one face associated with a caregiver on the video conference; receiving a trigger to transfer the video conference to a second device; authenticating the caregiver on the second device using the at least one face; and automatically transferring the video conference from the first device to the second device.

In another aspect, an example method for optimizing a video conference between a caregiver and a patient can include: initiating the video conference between the caregiver and the patient; determining an aspect of the video conference needs to be optimized; and performing optimization of the aspect of the video conference.

In yet another aspect, an example method of estimating an amount of time before a resource is allocated for a video call between a caregiver and a patient can include: receiving a request for the resource associated with the video call; calculating an estimated wait time for the resource to be available for the video call; and presenting the estimated wait time to one or more of the caregiver and the patient.

The invention will now be further described by way of example with reference to the accompanying drawings, in which:
FIG. 1 is a schematic diagram of a system that includes devices each operating a virtual care management application for managing consultations between a caregiver and remote care providers.
FIG. 2 illustrates an example method for using audio and/or video for patient care using the system of FIG. 1.
FIG. 3 illustrates an example of a sign in screen generated on a device of a caregiver by a virtual care management application of the system of FIG. 1.
FIG. 4 illustrates an example interface of the virtual care management application of FIG. 3 that includes a repository of videos captured by the caregiver.
FIG. 5 illustrates an example interface of the virtual care management application of FIG. 3 that delivers video.
FIG. 6 illustrates an example method for delivering video using the virtual care management application of FIG. 5.
FIG. 7 illustrates an example method for initiating a workflow on a device of a caregiver by a virtual care management application of the system of FIG. 1.
FIG. 8 illustrates an example interface of the virtual care management application of FIG. 7 that can be used to create or modify existing workflows.
FIG. 9 is a schematic diagram of another portion of the system of FIG. 1 that allows workflows to access resources external to a clinical care environment or the system.
FIG. 10 illustrates an example interface of the virtual care management application of FIG. 3 including a meeting room screen.
FIG. 11 illustrates an example method for transferring a video conference between devices of the system of FIG. 1.
FIG. 12 illustrates an example interface of the virtual care management application of FIG. 3 including aspects to optimize the video conference.
FIG. 13 illustrates an example interface of the virtual care management application of FIG. 3 including aspects to indicate wait times for resources for the video conference.
FIG. 14 schematically illustrates an example of a device from the system of FIG. 1 that can be used by a caregiver or remote care provider to implement aspects of the virtual care management application.

The present disclosure relates to the use of audio and/or video by caregivers to increase efficiencies in patient care. In general terms, audio and/or video is captured from a caregiver, and that audio and/or video is used to create greater efficiencies as the caregiver provides care to patients. Many different examples are provided below.

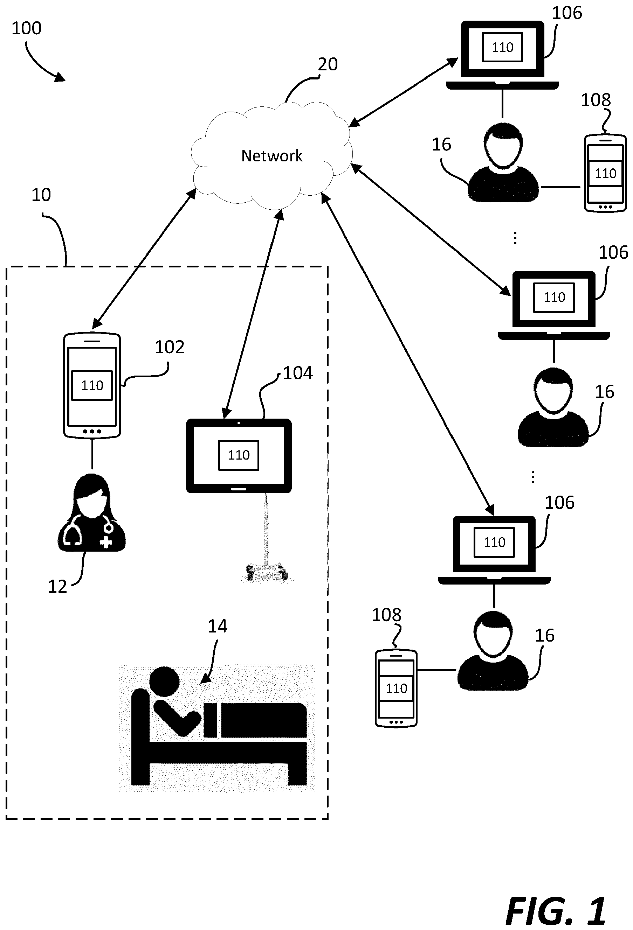

FIG. 1 is a schematic diagram of a system 100 that includes devices 102, 104, 106, 108 that each operate a virtual care management application 110 for managing consultations between a caregiver 12 and remote caregivers 16. As shown in FIG. 1, the caregiver 12 provides care to a patient 14 inside a clinical care environment 10. In some examples, the caregiver 12 is considered a local caregiver of the clinical care environment 10. In some further examples, the clinical care environment 10 is located in a rural, sparsely populated area.

As shown in FIG. 1, the remote caregivers 16 are located outside of the clinical care environment 10, and are remotely located with respect to the caregiver 12 and patient 14. As an illustrative example, the remote care providers can be located in a different city, county, or state from the location of the clinical care environment 10. Also, the remote caregivers 16 can be located remotely with respect to one another. For example, remote caregiver 16a can be located in a different city, county, or state than remote caregivers 16b, 16c.

In some examples, the remote caregivers 16 are medical specialists such as an intensivist, a neurologist, a cardiologist, a psychologist, and the like. In some further examples, a remote caregiver 16 is an interpreter/translator, or other kind of provider.

In certain examples, the virtual care management application 110 is installed on the devices 102, 104, 106, 108. Alternatively, the virtual care management application 110 can be a web-based or cloud-based application that is accessible on the devices 102, 104, 106, 108.

The virtual care management application 110 enables the caregiver 12 to provide acute care for the patient 14 by allowing the caregiver 12 to connect and consult with a remote caregiver 16 who is not physically located in the clinical care environment 10. Advantages for the patient 14 can include reducing the need to transfer the patient 14 to another clinical care environment or location, and minimizing patient deterioration through faster clinical intervention. Advantages for the caregiver 12 can include receiving mentorship and assistance with documentation and cosigning of medication administration. Advantages for the remote caregiver 16 can include allowing the remote caregiver 16 to cover more patients over a wider geographical area while working from a single, convenient location.

As shown in FIG. 1, the caregiver 12 can use both a primary device 102 and a secondary device 104 that each have the virtual care management application 110 installed thereon, or otherwise are able to access the virtual care management application 110 when hosted online or in a cloud computing network. In the example illustrated in the figures, the primary device 102 is a mobile device such as a smartphone that the caregiver 12 carries with them as they perform rounding and provide patient care in the clinical care environment 10.

The secondary device 104 can be a workstation such as a tablet computer, or a display monitor attached to a mobile stand that can be carted around the clinical care environment 10. The secondary device 104 can be shared with other caregivers in the clinical care environment 10. In some examples, the secondary device 104 can be a smart TV located in the patient's room, that is configured to access the virtual care management application 110.

The primary and secondary devices 102, 104 are interchangeable with one another. For example, in some alternative examples the secondary device 104 can be a smartphone carried by the caregiver 12, and the primary device 102 can be a workstation such as a tablet computer, a display monitor attached to a mobile stand, or a smart TV.

The remote caregivers 16 can similarly use both a primary device 106 and a secondary device 108 that can each access the virtual care management application 110. In the example illustrated in the figures, the primary device 106 of the remote caregiver 16 is a laptop, a tablet computer, or a desktop computer, and the secondary device 108 is a smartphone. The primary and secondary devices 106, 108 are interchangeable such that in some examples the secondary device 108 can be a laptop, a tablet computer, or a desktop computer, and the primary device 106 is a smartphone that the remote care provider carries with them.

The consultations between the caregiver 12 and the remote caregivers 16 are managed across a communications network 20. As shown in the example of FIG. 1, the primary and secondary devices 102, 104 used by the caregiver 12 are connected to the communications network 20, and the primary and secondary devices 106, 108 used by the remote caregivers 16 are also connected to the communications network 20. The communications network 20 can include any type of wired or wireless connections or any combinations thereof. Examples of wireless connections include broadband cellular network connections such as 4G or 5G.

A request from the caregiver 12 will go out to all remote caregivers 16 who have chosen to receive notifications for the request type and who are part of the health care system of the clinical care environment 10. Advantageously, the consultations between the caregiver 12 and the remote caregivers 16 are guided by the virtual care management application 110 to take the burden off the caregiver 12 to reach out to multiple care providers for a consultation. Instead, a request from the caregiver is sent to a plurality of remote care providers, and the remote care provider who accepts first gets connected to the caregiver who sent the request. This is achieved through combination of routing logic with a user activated interface. Advantageously, the virtual care management application 110 combines patient contextual data in a single application with communications and task management platforms.

Additionally, the virtual care management application 110 enables the remote caregivers 16 to cover multiple facilities within the health care system. Also, the virtual care management application 110 enables the remote caregivers 16 to select and change the type of notifications, request types, and facilities or units that they will receive notifications and virtual care requests on their devices from the virtual care management application 110.

Additional details regarding the system 100 can be found in U.S. Patent Application No. 63/166382 filed on March 26, 2021, the entirety of which is hereby incorporated by reference.

As described further in the examples provided below, one or more of the devices 102, 104, 106, 108 can be used to capture audio and/or video from the caregiver 12 and/or the remote caregivers 16 to enhance the delivery of patient care.

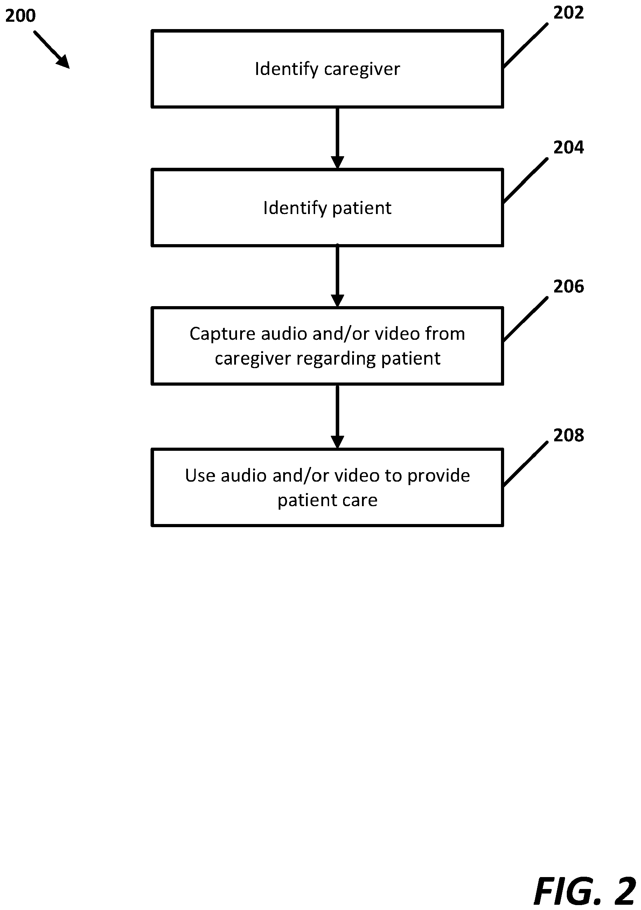

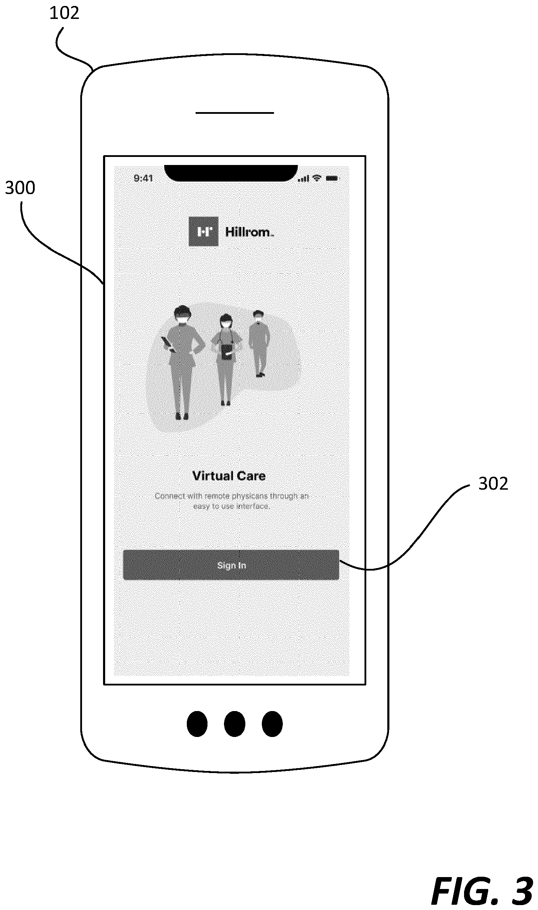

For example, referring now to FIG. 2, an example method 200 is shown for using audio and/or video for patient care. The method 200 includes an operation 202 of identifying the caregiver, which can involve some form of authentication by the caregiver (e.g., password, biometric, scan of badge/FOB, etc.). See FIG. 3 below. The authentication can be performed automatically based upon given criteria.

In an alternative embodiment, the caregiver can be authenticated, at least in part, using a Real Time Locating System (RTLS). The RTLS can be used to locate and/or identify the caregiver. One non-limiting example of such an RTLS is described in U.S. Patent Application Number 17/111075 filed on December 3, 2020, the entirety of which is hereby incorporated by reference.

Next, an operation 204 requires the identification of the patient for which the audio and/or video is directed. This can include a manual selection of a patient (e.g., by patient name or number) and/or automated selection of the patient based upon context (e.g., location or current assignment). As previously noted, an RTLS can also be used to located and/or authenticate the patient.

Next, at operation 206 audio and/or video is captured from the caregiver, and at operation 208 the captured audio and/or video is used in patient care. The patient care can include many different aspects of patient care, including communication between the caregiver and other caregivers and/or the patient, workflow implementations, and the like. Each of the operations 206 and 208 will be described in more detail with respect to the various embodiments described below.

FIG. 3 illustrates an example of a sign in screen 300 generated on the primary device 102 of the caregiver 12 by the virtual care management application 110. The sign in screen 300 shown in FIG. 3 can be used by the caregiver 12 to perform the operation 202 of signing into the virtual care management application 110, in accordance with the method 200 that is described above. The sign in screen 300 can be automatically displayed when the caregiver 12 opens the virtual care management application 110 on their primary device. The sign in screen 300 includes a sign in icon 302 that the caregiver 12 can select to sign into the virtual care management application 110.

Upon signing in, the primary device 102 of the caregiver 12 is configured to capture audio and/or video from the caregiver. As is typical in mobile devices, the primary device 102 can include at least one microphone to capture the audio from the caregiver 12 and at least one camera to capture photographs and/or video from the caregiver 12.

In some examples provided herein, the audio and/or video from the caregiver 12 is captured and recorded. In other examples, the audio and/or video from the caregiver 12 is captured and delivered to another, such as the patient 14, to allow for a two-way communication between the caregiver 12 and the patient 14. Many of these configurations are described below.

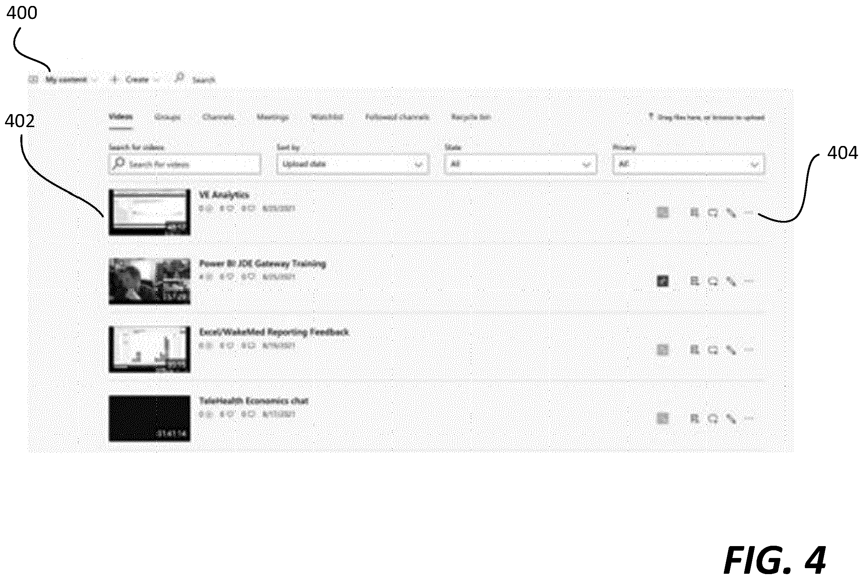

Referring now to FIGS. 4-6, one example of the capture of audio and/or video is provided. In this example, the caregiver can capture audio and/or video when transitioning care to another caregiver and/or during discharge of the patient. Such a process is common when a caregiver is ending a shift and must transfer information about the patient to the next caregiver. Additionally, the caregiver can provide information to the patient and/or the patient's family.

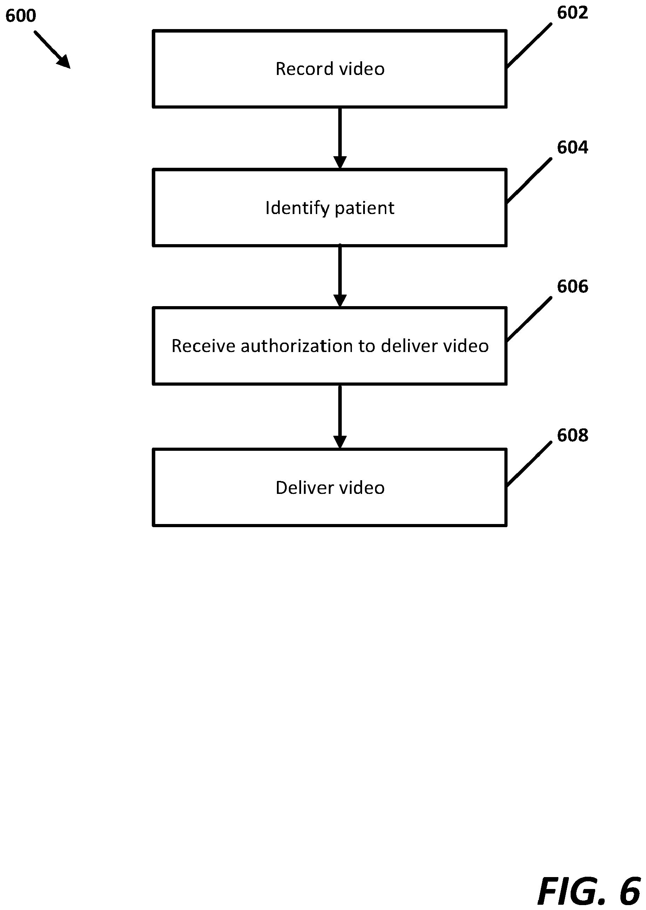

In this example, the primary device 102 of the caregiver 12 is used to capture video from the caregiver 12 about that transition in care. As shown in FIG. 4, an interface 400 provided by the virtual care management application 110 includes a repository of the videos captured by the caregiver 12. Once a video is captured (see FIG. 6 below), the video is displayed in a list 402 of the interface 400. The caregiver 12 can access the videos and play, delete, replace, and/or deliver the videos as desired using controls 404 of the interface.

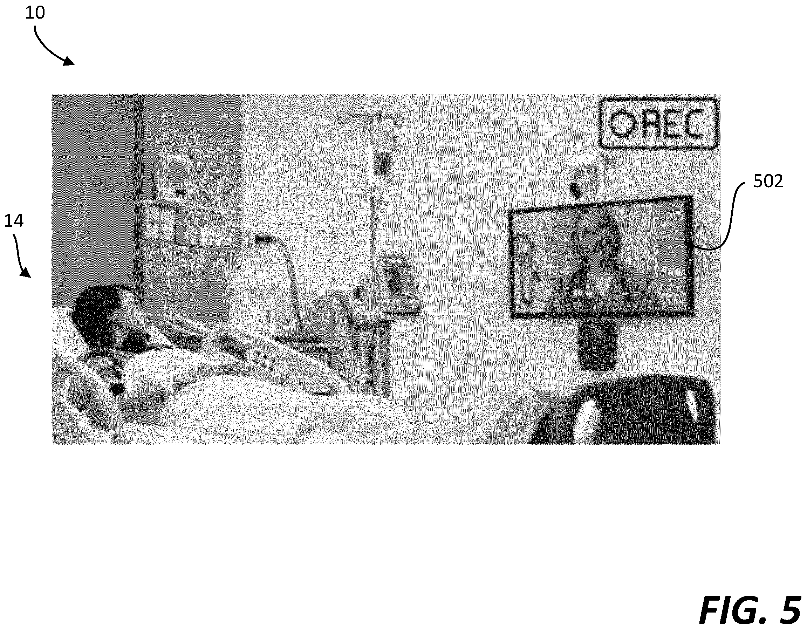

Referring now to FIG. 5, an example of delivery of the video to the patient 14 is shown. In this example, the recorded video is played on a playback device 502, such as a computer or television located in the clinical care environment 10, in this instance the room of the patient 14.

Referring now to FIG. 6, an example method 600 for delivering video as shown in FIGS. 4-5 is provided. At operation 602, the primary device 102 captures a video from the caregiver 12. As noted, this video can relate to some aspect of patient care and can be for the consumption of the next caregiver, the patient 14, and/or the family of the patient 14. Next, at operation 604, primary device 102 receives a selection of the patient 14. As noted, the selection of the patient 14 can be manual or automated by the primary device 102.

Next, at operation 606 the primary device 102 receives authorization to deliver the video, and at operation 608 the video is delivered.

In the example above, the video can be delivered to the patient 14 to provide the patient 14 with information about the care of the patient 14 during transition from the caregiver 12 to a subsequent caregiver. In other examples, the video can be delivered to the next caregiver and/or the patient of the caregiver.

In some examples, the primary device 102 can receive instructions from the caregiver 12 for routing and delivery of the video. For instance, the caregiver 12 can record a single video to be delivered to both the patient 14 and the next caregiver or record different videos for delivery to each.

In some examples, the virtual care management application 110 can automate the routing and delivery of the video to the appropriate parties.

For example, the virtual care management application 110 can be programmed to automate the delivery of the video to a chatroom associated with the patient. Additional details on these chatrooms are provided in U.S. Patent Application No. 17/453273 filed on November 2, 2021, the entirety of which is hereby incorporated by reference.

Additional details regarding delivery of messages, including the audio and/or video described herein, to patient families is provided in U.S. Patent Application No. 63/163468 filed on March 19, 2021, the entirety of which is hereby incorporated by reference.

Additional details regarding delivery of care instructions including the audio and/or video described herein, across different aspects of patient care within the system 100 (as well as possibly within the home of the patient) are provided in U.S. Patent Application No. 63/362250 (Attorney Docket 14256.0060USP1) filed on March 31, 2022, the entirety of which is hereby incorporated by reference.

There are various other aspects that can be associated with the capture of the audio and/or video from the caregiver. For instance, the audio and/or video can be transcribed to create a text version. In other examples, the audio can automatically be translated, especially if the patient 14 or the family speaks a different language. This can again be done in text or audio formats. Finally, the audio and/or video can be used for documentation purposes and captured in, for example, the Electronic Medical Record (EMR) associated with the patient.

In other examples, a prompt can automatically be provided to the caregiver 12 at desired intervals to capture the audio and/or video. For instance, when the caregiver 12 is getting ready to end a shift, the virtual care management application 110 can be programmed to automatically prompt the caregiver 12 to capture audio and/or video associated with the handoff. Similarly, when the caregiver 12 provides discharge instructions, the virtual care management application 110 can be programmed to automatically capture audio and/or video from the caregiver 12 associated with the discharge.

The delivery of the video can enhance the system 100 by allowing the caregiver 12 to deliver the information more efficiently to the various parties. For instance, the caregiver 12 may not be located in an area where the caregiver 12 can easily access the next caregiver or the patient 14, so delivering the video to the next caregiver or patient 14 is more efficient because the caregiver 12 does not need to locate the next caregiver or the patient 14. Further, the caregiver 12 can record and deliver multiple videos quickly, thereby allowing the caregiver 12 to deliver the required information more efficiently than having to walk around the care facility to greet each caregiver and patient individually. This can help to reduce the inefficiencies associated with the exchange of information and errors associated therewith. Other advantages are possible.

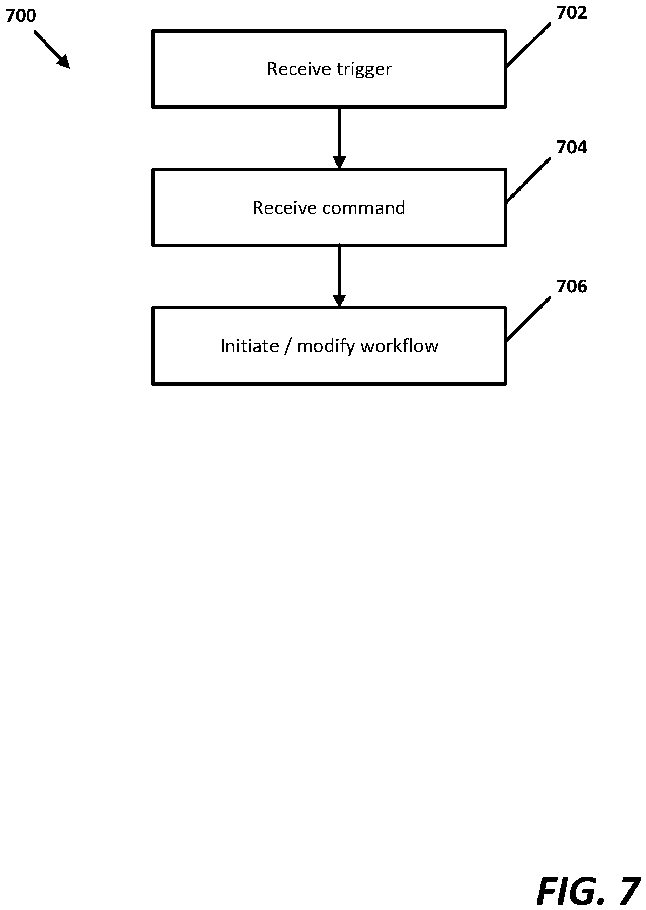

In addition to the capturing audio and/or video for delivery to others, the system 100 can capture audio and/or video to initiate or modify existing workflows associated with the care of the patient 14. For instance, referring now to FIGS. 7-9, examples of capturing audio and/or video are shown that can trigger workflows or modify existing workflow.

In the examples provided herein, a workflow is one or more actions associated with the care of the patient 14. Examples of such workflows include prescribing a drug for a patient, initiating a ventilator for a patient, a consult (in-person or virtual), etc.

In these examples, a workflow can be initiated or modified based upon audio and/or video captured from the caregiver 12. For instance, referring to FIG. 7, an example method 700 for initiating a workflow is provided. At operation 702, a trigger is received from the primary device 102 of the caregiver 12. This trigger can be audible, such as by the caregiver 12 uttering a keyword or phrase. In other instances, the trigger can be physical, such as receipt of a button press on the primary device 102 of the caregiver 12 or a gesture captured by the primary device 102 of the caregiver 12.

Once a trigger is received, the primary device 102 of the caregiver 12 monitors or otherwise waits for and receives a command from the caregiver 12 at operation 704. The command can be verbalized by the caregiver 12, for instance: "Initiate ventilation". In other scenarios, the command can be received through other methods, such as from a gesture by the caregiver 12.

Finally, at operation 706, the primary device 102 of the caregiver 12 initiates or modifies a given workflow based upon the command from the caregiver 12. For example, in the instance of the command "Initiate ventilation", the primary device 102 can implement a ventilation workflow that gathers the necessary resources to ventilate the patient 14, including the ventilator, personnel to deliver and initiate the ventilation, and any other requirements for the ventilation workflow. Further, context associated with issuance of the command can be used.

For instance, the primary device 102 can be location-aware (e.g., Real-Time Locating Systems (RTLS)), so that when the caregiver 12 issues a command in a particular room, the workflow is initiated by the primary device 102 for the patient associated with that room. One example of a system using such an RTLS is disclosed in U.S. Patent Application No. 17/111,075 filed on December 3, 2020, the entirety of which is hereby incorporated by reference.

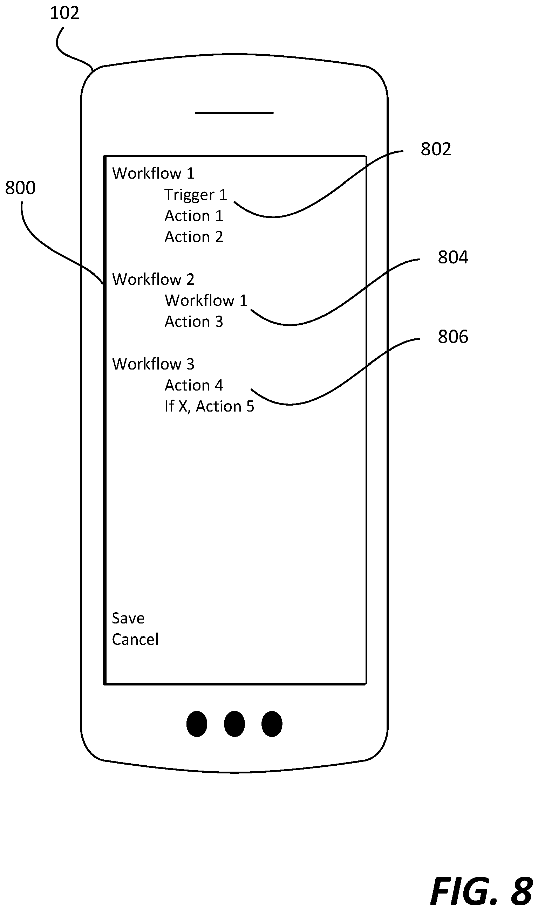

Referring now to FIG. 8, an example interface 800 of the primary device 102 is shown. The interface 800 is configured to allow the caregiver 12 to create or modify existing workflows 802, 804, 806. For instance, the workflow 802 includes a name ("Workflow 1") that can characterize what the workflow does. For instance, the Workflow 1 can be tagged with the name "Ventilator". The workflow 802 also includes a trigger, which is the audible set of words that are used to trigger the workflow. In this example, the trigger can be "Initiate ventilator". Finally, various actions are associated with the workflow. Example actions can include, for instance, placing a work order for the ventilator and contacting one or more personnel who will connect the ventilator to the patient. Further, the system 100 can be programmed to route notifications about the action to the appropriate caregivers, such as through integration with a scheduling system that indicates which caregivers are currently working for a given shift or period of time.

The actions can be configurable and put together like building blocks to create the desired workflows. For instance, the workflows can be nested (see, e.g., workflow 804) and put together from existing actions to assist in their creation. In some examples, the workflows can be defined by the caregiver 12 and/or include pre-defined workflows defined for the system 100. Further, the caregiver 12 can use the interface 800 to modify the workflows as desired. Many configurations are possible.

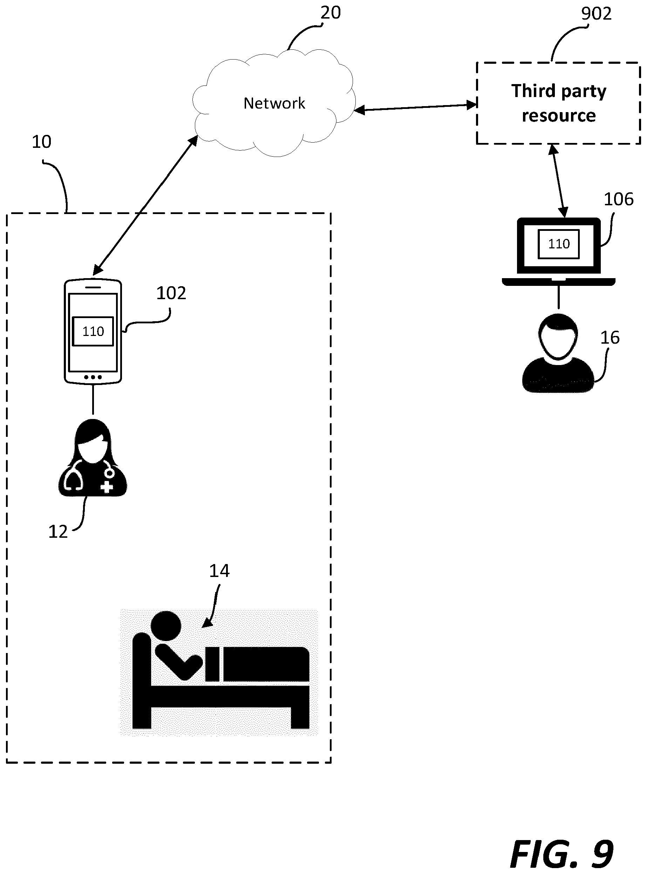

Referring now to FIG. 9, in some examples the workflows can be used to access resources external to the clinical care environment 10 or even the system 100. For instance, once initiated, a workflow on the primary device 102 of the caregiver 12 can access resources remote from the clinical care environment 10. This can be accomplished, for instance, through an Application Programming Interface (API) associated with a third party resource 902.

For instance, if a workflow requires a specialty consult by the remote caregiver 16, the workflow can automatically initiate a call to the third party resource 902. The third party resource 902 can, in turn, manage connection of the caregiver 12 to the remote caregiver 16 at the clinical care environment 10 for a virtual consult. Many other configurations are possible.

In some examples, the workflows can provide updates to the various records associated with the patient 14. For instance, with the example relating to the ventilator, the workflow can update the chatroom associated with the patient 14 to indicate that ventilation has been ordered and also provide updates as the ventilator is delivered and initiated. The workflow can further highlight certain aspects of the entries in the chatroom that may be important or otherwise require action by the caregiver 12.

Although the examples provided discuss the initiation of a workflow, the examples can also be used to modify a workflow or stop a workflow. For instance, the trigger can be used to modify an existing workflow or provide input for the workflow. For example, if a workflow requires a particular parameter to execute, the workflow can receive that parameter from the caregiver through further input from the caregiver. Similarly, the input from the caregiver can be received to stop a workflow or substitute one workflow for another. Many configurations are possible.

As noted, video can also be captured along with or in place of audio to initiate or modify workflows. For instance, gestures rather than audio input can be received from the caregiver to initiate a particular workflow.

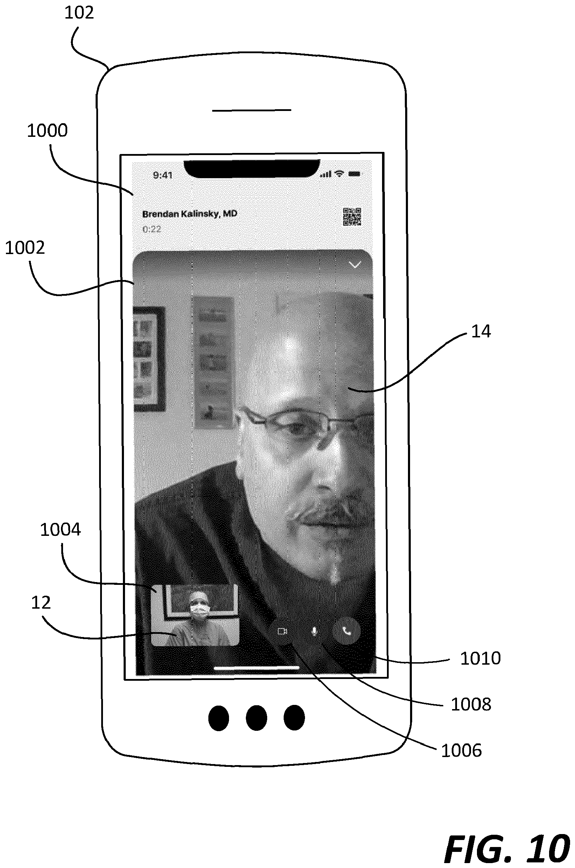

Referring now to FIGS. 10-11, in some examples the audio and/or video captured from the caregiver 12 is delivered to another party so that two-way communication can be initiated. For instance, the caregiver 12 can use the primary device 102 to capture audio and/or video and communicate directly with the patient 14, as illustrated in FIG. 10.

FIG. 10 illustrates an example of a meeting room screen 1000 that is generated on the primary device 102 of the caregiver 12 by the virtual care management application 110 in response to the caregiver 12 initiating the video conference. The meeting room screen 1000 can display the name of the patient 14 and can also display a duration of the meeting. The meeting room screen 1000 further includes a window 1002 that displays a live video feed of the patient 14 who accepted the care request. The caregiver 12 can communicate with the patient 14 using the audio system of the primary device 102 (i.e., speakers and microphone), while viewing the patient 14 in the window 1002 such that the meeting room screen 1000 provides at least a one-way video conference with the patient 14.

The meeting room screen 1000 can further include a window 1004 that displays a live video feed of the caregiver 12 acquired from the camera of the primary device 102. In such examples, the meeting room screen 1000 can provide a two-way video conference between the caregiver 12 and patient 14. The meeting room screen 1000 can include a video camera icon 1006 that the caregiver can select to turn on and off the camera of the primary device 102, and thereby allow or block the live video feed of the caregiver 12. The meeting room screen 1000 can also include a microphone icon 1008 that the caregiver 12 can select to turn off and on the microphone of the primary device 102, and thereby mute and unmute the caregiver 12. The meeting room screen 1000 can also include a hang up icon 1010 that the caregiver 12 can select to terminate the video conference with the patient 14.

To initiate such a conference, the caregiver 12 can be authenticated on the primary device 102 (e.g., through a password, biometrics, FOB/scanner, etc.). Upon authentication, the caregiver 12 can initiate the video conference with the patient 14 by selecting the patient from a list, selecting a specific room, etc. The patient 14 can communicate with a device located in the room of the patient 14 or possibly a personal device of the patient 14.

When this conference is happening between the caregiver 12 and the patient 14, the caregiver 12 and patient can discuss any desired topics, such as the care of the patient, changes in that care, etc. As the discussion is occurring, the caregiver 12 may wish to change the device used to conduct the conference.

For instance, the caregiver 12 may initiate the conference on the primary device 102 while the caregiver 12 is moving. The caregiver 12 may then reach a place where the caregiver 12 has another device that may be more conducive or easier to use, such as the secondary device 104. The secondary device 104 can be a display monitor attached to a mobile stand that can be carted around the clinical care environment 10. Upon reaching the secondary device 104, the video conference with the patient 14 can automatically be transferred to the secondary device 104 from the primary device 102 to allow the caregiver 12 more flexibility, such as not having to hold the primary device 102.

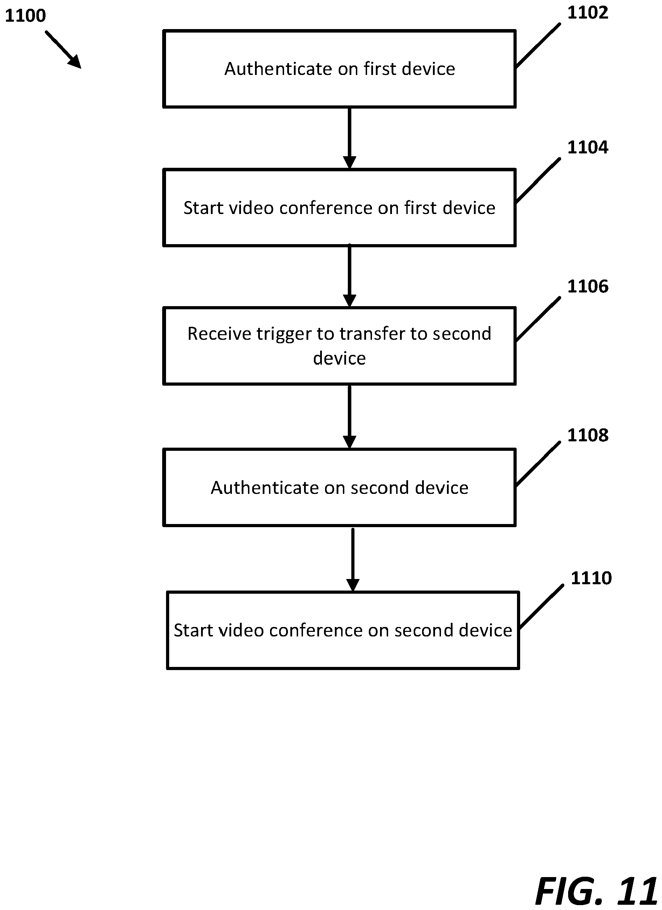

More specifically, FIG. 11 shows an example method 1100 for transferring the video conference. At operation 1102, the caregiver 12 is authenticated on a first device (e.g., the primary device 102). As noted, this authentication can be done many ways, such as with a PIN, password, biometrics, scan of badge/FOB, etc. Once authenticated, the first device can initiate the video conference with the patient 14 at operation 1104.

Next, at operation 1106, either the first device or a second device (e.g., the secondary device 104) received a trigger to transfer the video conference to the second device. This trigger can be manual, such as through a request received from the caregiver 12 on the first device or the second device. The trigger can be automated, in that a prompt (e.g., toast or other notification) is presented on the first device when the first device is within a specific distance of the second device, such as a few feet. In yet another example, the trigger can simply be entering the field of view of another camera, such as entering the view of the camera on the secondary device 104.

In either event, when the transfer is initiated, the caregiver 12 is authenticated on the second device at operation 1108. In some examples, this authentication can happen automatically, such as by recognizing the face of the caregiver 12 on the second device using facial recognition. For instance, the caregiver 12 can simply present his or her face to the camera of the second device, and the second device can use the face to authenticate the caregiver 12. In one example, the first device uses facial recognition to identify the face or faces in the field of view of the first device. Upon one of more of those faces being identified in the field of view of a camera on the second device, the second device can automatically authenticate the face.

Finally, at operation 1110, the video conference is transferred to the second device upon authentication.

A similar transition can occur should the caregiver 12 enter the room of the patient 14 while a video conference is occurring. For example, the caregiver 12 can initiate a video conference with the patient 14 as the caregiver 12 is enroute to the room of the patient 14. This allows the caregiver 12 to begin conveying information to the patient 14 even before the caregiver 12 arrives physically in the room, thereby increasing efficiency.

When the caregiver 12 reaches a close proximity to the patient 14 (e.g., 20 feet, 10 feet, 5 feet, or enters the room of the patient 14), the primary device 102 can be programmed to automatically end the video conference, since the caregiver 12 is now in physical proximity to the patient 14 and the video conference is no longer needed. For example, the primary device 102 can use location information (e.g., GPS, RTLS) or other data (e.g., RFID beacons) to determine that the caregiver 12 has entered the room of the patient 14 and automatically end the video conference.

The transitions can occur for multiple providers when the video conference involves more than two individuals. These examples help to automate the transitions associated with video conferencing between the caregiver 12 and the patient 14. Ideally, the transitions become less intrusive to both and provide a seamless ability for communication. Many other configurations are possible.

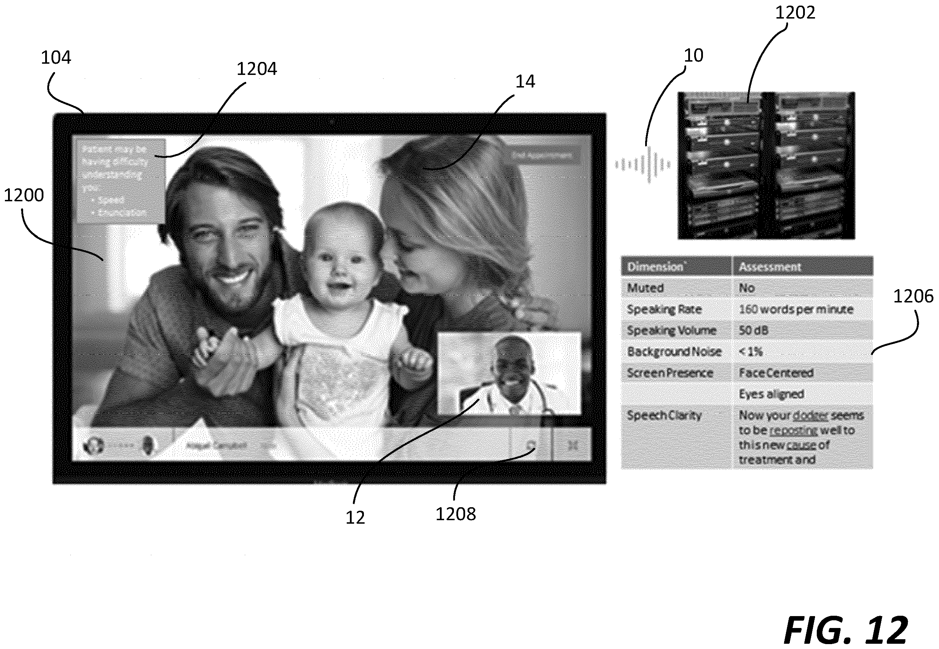

Referring now to FIG. 12, in addition to facilitating transfer of the video conference between devices, examples provided herein can assist in optimizing the experience in a video conference between the caregiver 12 and the patient 14.

More specifically, the caregiver 12 can use a device, such as the primary device 102 and/or the secondary device 104, to conduct a video conference with the patient (or patients) 14, as described previously. In such a scenario, a display 1200 provides the video feed, and one or more microphones and speakers of the secondary device 104 allow the caregiver 12 to communicate with the patient 14.

During the video conference, the secondary device 104 or a server 1202 facilitating the video conference can be programmed to optimize the communication between the caregiver 12 and the patient 14. For instance, the server 1202 can automatically analyze the audio and/or video associated with the video conference and make recommendations or reconfigurations to optimize the video conference.

In the example, the server 1202 analyzes the speech of the caregiver 12 and makes recommendations to optimize the likelihood that the patient 14 can understand the caregiver. In the example, the server 1202 creates a pop-up window 1204 that provides recommendations to the caregiver 12, such as to slow the speed of their speech and better enunciate their spoken words. This can be created based upon an analysis of the audio feed from the secondary device 104 of the caregiver 12.

Further, the server 1202 can analyze the conditions for the patient 14 and provide recommendations and/or optimizations to the caregiver 12 and/or the patient 14. For instance, the server 1202 can generate a window 1206 that provide metrics associated with the video conference between the caregiver 12 and the patient 14, such as whether the patient is muted, the speaking rate, the volume, background noise, screen presence, and speech clarity.

If there are issues with any of the metrics, the server 1202 can provide recommendations to fix the possible issue and/or automatically do so. For instance, if the speech rate is too fast, the server 1202 can generate the pop-up window 1204 described above. Additional examples can include, without limitation, the following:
speaking rate - if the speaker (caregiver 12 and/or patient 14) is exceeding a suggested words-per-minute, suggest that they slow their speech;
clarity of speech - use of voice recognition technology to judge whether speech can be successfully converted to (intelligible) text and, if not, notify the speaker that they may need to focus on more clearly enunciating their words;
volume - system alert when voice volume below pre-defined minimum level (for example, when mouth not close enough to microphone);
background noise - system alert when recognizing that background noise (for example, by recognizing non-vocal sounds, sounds not correlating to mouth movements);
screen presence - system alert (e.g., on-screen reminder, vocal cue) if a speaker is not positioned in front of the camera correctly (face not in view, not making eye contact); and
pronoun correction - given the sensitivities regarding use of somebody's preferred gender pronoun (e.g., she / her / hers, he / him / his, they / them / theirs), recognize if the caregiver 12 is not using preferred pronouns for the patient 14.

Further, if the server 1202 senses that the patient 14 is trying to talk (either through audio and/or video analysis showing the lips of the patient 14 moving) but is on mute, the server 1202 can indicate such to the caregiver 12 and/or the patient 14 or simply automatically unmute the patient 14. In addition, if the background noise increases, the sound from the speakers for the caregiver 12 and/or the patient 14 can be increased (and/or noise cancelation can be turned on or off). Further, if the face of the caregiver 12 and/or the patient 14 is not centered in the camera view, the server 1202 (or local device) can recenter the image as necessary to optimize the view. Many other configurations are possible.

In addition, the server 1202 can be programmed to optimize the language used for communication between the caregiver 12 and the patient 14. For instance, a language preference can be captured at the beginning of the video conference or language can be automatically detected during conversation on the video conference.

If the server 1202 identifies a disconnect between the language of the caregiver 12 and the patient 14, the server 1202 can either provide automatic translation of the language or request an interpreter.

If auto-translation is provided, once the languages are identified there can be an automatic translation of voice and text to the appropriate language using, for instance, artificial intelligence. The caregiver 12 and/or the patient 14 can indicate gaps in understanding (or translation issues) via a button 1208 on the display 1200. This will enable additional training of the algorithm as needed. Audio transcripts can also be sent over to native speaking auditors to ensure all of the details of the encounter are understood and properly translated.

If an interpreter is needed, the server 1202 can automatically request the interpreter and facilitate the conference of the interpreter with the existing video call between the caregiver 12 and the patient 14.

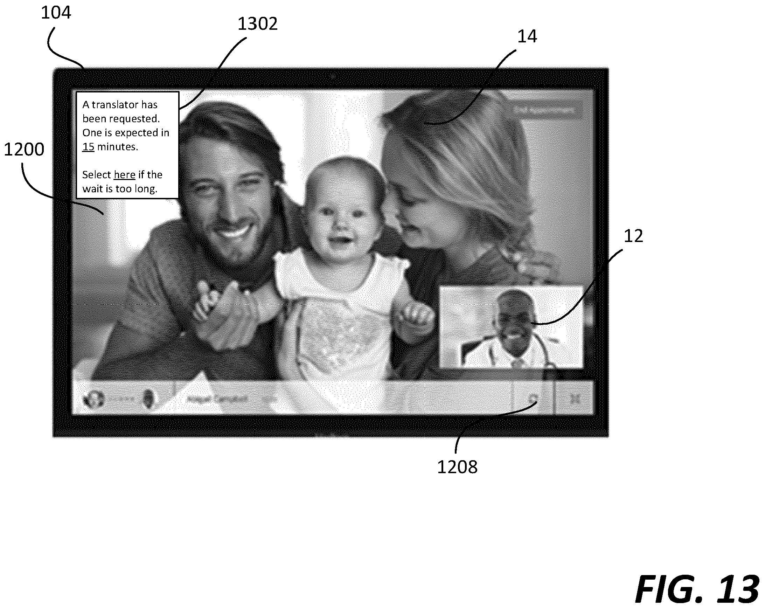

As resources such as the translator are requested, it can be desirable to provide an indication to the caregiver 12 and/or the patient 14 regarding the availability of the resources. Referring now to FIG. 13, in some examples artificial intelligence is used to predict the response times of a requested resource and gives an indication back to the requestor of the expected wait time.

In this example, when an interpreter is requested, the server 1202 provides a pop-up window 1302 indicating that the resource has been requested and an estimated time for the resource to be available. In this instance, the server 1202 estimates that the interpreter will be available in 15 minutes. Although this example includes the translator as the resource, many other types of resources can be requested. For instance, a remote specialist in a particular area of medicine is an example of another type of resource that can be requested.

In order to provide the estimate, the server 1202 uses artificial intelligence, such as machine learning, to develop an algorithm to estimate when resources are likely to be available. The algorithm can look at one or more of the following when determining likely response times:
number of facilities that resource is covering on a shift;
if the resource is fully remote for the shift or on site at a facility and covering others;
number of resources on shift for given request types;
average wait time for that resource, broken down by priority of the request;
average wait time for low, med, high priority calls for that resource;
average call length for that resource, broken down by priority of the request;
average call length for low, med, high priority calls; and
number of requests pending in the queue for that resource.

These are just some of the examples of the types of inputs that can be provided. The algorithm, as developed, looks at the request for the translator and provides an estimate of the amount of time until the translator is available.

A link is also provided in the window 1302 should the delay be excessive or otherwise unacceptable. If so, the caregiver 12 or the patient 14 can select the link to escalate the request. For instance, when a resource is requested, the initial request can be indicated as low priority (or escalated immediately based upon the context, such as type of resource, patient condition, etc.). Should the amount of time to wait for the resource be excessive, accessing the link will allow the caregiver 12 to raise the priority level of the request for the resource. This will escalate the request and allow the resource to be allocated more quickly. Many other configurations are possible.

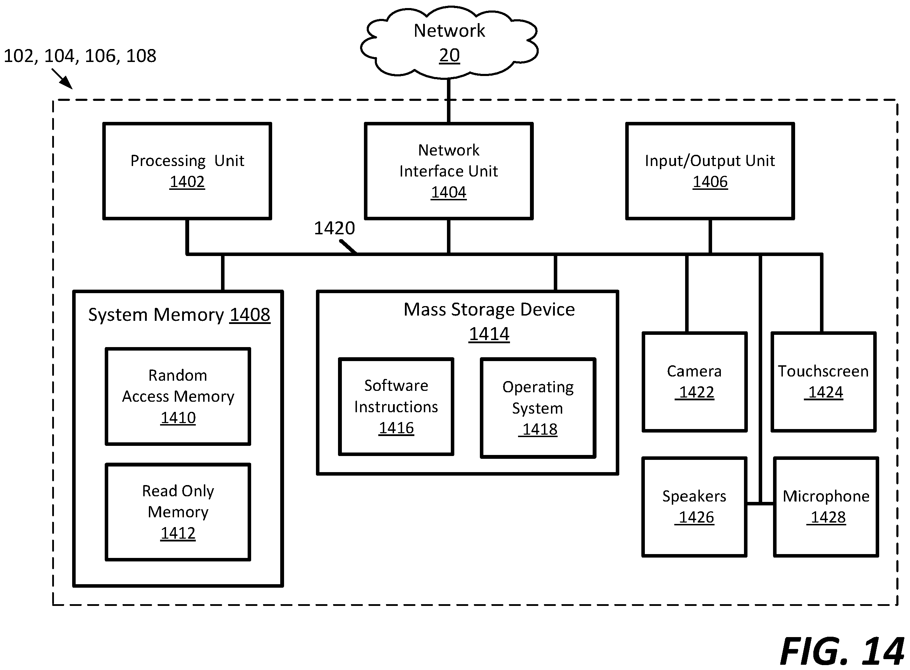

FIG. 14 schematically illustrates in more detail an example of the device 102, 104, 106, 108 that can be used by the caregiver 12 and/or the patient 14 to implement aspects of the virtual care management application 110. The device 102, 104, 106, 108 includes a processing unit 1402, a system memory 1408, and a system bus 1420 that couples the system memory 1408 to the processing unit 1402. The processing unit 1402 is an example of a processing device such as a central processing unit (CPU). The system memory 1408 includes a random-access memory ("RAM") 1410 and a read-only memory ("ROM") 1412. A basic input/output logic having basic routines that help to transfer information between elements within the device 102, 104, 106, 108, such as during startup, is stored in the ROM 1412.

The device 102, 104, 106, 108 can also include a mass storage device 1414 that is able to store software instructions and data. The mass storage device 1414 is connected to the processing unit 1402 through a mass storage controller (not shown) connected to the system bus 1420. The mass storage device 1414 and its associated computer-readable data storage media provide non-volatile, non-transitory storage for the device 102, 104, 106, 108.

Although the description of computer-readable data storage media contained herein refers to a mass storage device, it should be appreciated by those skilled in the art that computer-readable data storage media can be any available non-transitory, physical device or article of manufacture from which the device can read data and/or instructions. In certain embodiments, the computer-readable storage media comprises entirely non-transitory media. The mass storage device 1414 is an example of a computer-readable storage device.

Computer-readable data storage media include volatile and non-volatile, removable and non-removable media implemented in any method or technology for storage of information such as computer-readable software instructions, data structures, program modules or other data. Example types of computer-readable data storage media include, but are not limited to, RAM, ROM, EPROM, EEPROM, flash memory or other solid-state memory technology, or any other medium which can be used to store information, and which can be accessed by the device.

The device 102, 104, 106, 108 operates in a networked environment using logical connections to devices through the communications network 20. The device 102, 104, 106, 108 connects to the communications network 20 through a network interface unit 1404 connected to the system bus 1420. The network interface unit 1404 can also connect to additional types of communications networks and devices, including through Bluetooth, Wi-Fi, and cellular.

The network interface unit 1404 may also connect the device 102, 104, 106, 108 to additional networks, systems, and devices such as a digital health gateway, electronic medical record (EMR) system, vital signs monitoring devices, and clinical resource centers.

The device 102, 104, 106, 108 can also include an input/output unit 1406 for receiving and processing inputs and outputs from a number of peripheral devices. Examples of peripheral devices may include, without limitation, a camera 1422, a touchscreen 1424, speakers 1426, a microphone 1428, and similar devices used for voice and video communications.

The mass storage device 1414 and the RAM 1410 can store software instructions and data. The software instructions can include an operating system 1418 suitable for controlling the operation of the device 102, 104, 106, 108. The mass storage device 1414 and/or the RAM 1410 also store software instructions 1416, that when executed by the processing unit 1402, cause the device to provide the functionality of the device 102, 104, 106, 108 discussed herein.

The various embodiments described above are provided by way of illustration only and should not be construed to be limiting in any way. Various modifications can be made to the embodiments described above without departing from the true spirit and scope of the disclosure.

Aspects of the present disclosure are defined in the following numbered clauses:
1. A method for delivery of patient information through audio and/or video, the method comprising:
   capturing audio and/or video from a caregiver;
   receiving identification of a patient from the caregiver;
   receiving authorization to deliver the audio and/or video in association with providing care for the patient; and
   delivering the audio and/or the video.
2. The method of clause 1, further comprising delivering the audio and/or the video to one or more of a family member of the patient and a subsequent caregiver of the patient.
3. The method of clause 1 or 2, further comprising automatically prompting the caregiver to capture the audio and/or video upon handoff or discharge of the patient.
4. The method of clause 1, 2, or 3, further comprising automatically transcribing and translating the audio and/or the video.
5. The method of any preceding claim, further comprising:
   receiving a trigger event;
   upon receiving the trigger event, monitor for a command from the caregiver of the patient; and
   upon receiving the command, initiate a workflow associated with the command.
6. A method for initiating a workflow for a patient, the method comprising:
   receiving a trigger event;
   upon receiving the trigger event, monitor for a command from a caregiver of the patient; and
   upon receiving the command, initiate the workflow associated with the command.
7. The method of clause 5 or 6, wherein the trigger event is a keyword or phrase.
8. The method of clause 5, 6, or 7, wherein the command is a phrase identifying the workflow.
9. The method of any of clauses 5 to 8, wherein the workflow is associated with care of the patient.
10. The method of any of clauses 5 to 9, further comprising allowing the caregiver to build the workflow.
11. The method of any of clauses 5 to 10, further comprising accessing an external resource as part of the workflow.
12. A method for conducting a video conference associated with care of a patient, the method comprising:
   initiating the video conference on a first device;
   identifying at least one face associated with a caregiver on the video conference;
   receiving a trigger to transfer the video conference to a second device;
   authenticating the caregiver on the second device using the at least one face; and
   automatically transferring the video conference from the first device to the second device.
13. The method of clause 12, further comprising identifying the at least one face using facial recognition.
14. The method of clause 12 or 13, further comprising automatically transferring the video conference to the second device when the at least one face is within a field of view of the second device.
15. The method of clause 12, 13, or 14, further comprising automatically ending the video conference when the caregiver is in close proximity to the patient.
16. The method of clause 15, further comprising using a location of the first device to determine the close proximity.
17. A method for optimizing a video conference between a caregiver and a patient, the method comprising:
   initiating the video conference between the caregiver and the patient;
   determining an aspect of the video conference needs to be optimized; and
   performing optimization of the aspect of the video conference.
18. The method of clause 17, further comprising presenting a window indicating the aspect to be optimized to the caregiver.
19. The method of clause 17 or 18, wherein the aspect is one or more of speaking rate, clarity of speech, volume, background noise, screen presence, and pronoun usage.
20. The method of clause 17, 18, or 19, further comprising providing automated translation of speech of the caregiver or the patient.
21. The method of any of clauses 17 to 19, further comprising:
   detecting a disconnect in a language between the caregiver and the patient;
   automatically requesting an interpreter; and
   adding the interpreter to the video conference to provide interpretation services.
22. A method of estimating an amount of time before a resource is allocated for a video call between a caregiver and a patient, the method comprising:
   receiving a request for the resource associated with the video call;
   calculating an estimated wait time for the resource to be available for the video call; and
   presenting the estimated wait time to one or more of the caregiver and the patient.
23. The method of clause 22, further comprising using artificial intelligence to calculate the estimated wait time.
24. The method of clause 22 or 23, further comprising creating an algorithm to calculate the estimated wait time, wherein the algorithm uses one or more of: number of resources on shift for the resource; average wait time for the resource; average video conference call length for the resource; and number of requests pending for the resource.
25. The method of clause 22, 23, or 24, further comprising allowing the caregiver to prioritize the request for the resource.
26. A system, comprising:
   at least one processor; and
   memory encoding instructions that, when executed by the at least one processor, cause the system to:
      capture audio and/or video from a caregiver;
      receive identification of a patient from the caregiver;
      receive authorization to deliver the audio and/or video in association with providing care for the patient; and
      deliver the audio and/or the video.

## Claims

1. A method for conducting a video conference associated with care of a patient, the method comprising:
initiating the video conference on a first device;
identifying at least one face associated with a caregiver on the video conference;
receiving a trigger to transfer the video conference to a second device;
authenticating the caregiver on the second device using the at least one face; and
automatically transferring the video conference from the first device to the second device.

2. The method of claim 1, wherein the first device is a mobile device, and wherein the second device is a workstation.

3. The method of claim 1 or 2, further comprising, prior to initiating the video conference, authenticating the caregiver on the first device.

4. The method of claim 1, 2, or 3, further comprising:
identifying the at least one face using facial recognition; and
automatically transferring the video conference to the second device when the at least one face is within a field of view of the second device.

5. The method of any preceding claim, further comprising automatically ending the video conference when the caregiver is in close proximity to the patient.

6. The method of any preceding claim, wherein the trigger is received by the first device, or by the second device.

7. The method of any preceding claim, wherein the trigger is a manual trigger, or wherein the trigger is an automated trigger.

8. The method of claim 7, wherein the trigger is an automated trigger, and wherein the method further comprises: presenting a prompt on the first device when the first device is within a specific distance to the second device.

9. The method of any preceding claim, further comprising:
initiating the video conference between the caregiver and the patient;
determining an aspect of the video conference needs to be optimized; and
performing optimization of the aspect of the video conference.

10. The method of claim 9, further comprising presenting a window indicating the aspect to be optimized to the caregiver, wherein the aspect is one or more of speaking rate, clarity of speech, volume, background noise, screen presence, and pronoun usage.

11. The method of any preceding claim, further comprising providing automated translation of speech of the caregiver or the patient.

12. The method of any of claims 1 to 10, further comprising:
detecting a disconnect in a language between the caregiver and the patient;
automatically requesting an interpreter; and
adding the interpreter to the video conference to provide interpretation services.

13. The method of any preceding claim, further comprising estimating an amount of time before a resource is allocated for a video call between a caregiver and a patient, wherein estimating an amount of time before a resource is allocated for a video call between a caregiver and a patient comprises:
receiving a request for the resource associated with the video call;
calculating an estimated wait time for the resource to be available for the video call; and
presenting the estimated wait time to one or more of the caregiver and the patient.

14. The method of claim 13, wherein estimating an amount of time before a resource is allocated for a video call between a caregiver and a patient further comprises: creating an algorithm to calculate the estimated wait time, wherein the algorithm uses one or more of: number of resources on shift for the resource; average wait time for the resource; average video conference call length for the resource; and number of requests pending for the resource.
